# EUROPÄISCHE PATENTANMELDUNG

(11) **EP 4 086 547 A1**
(43) Veröffentlichungstag der Anmeldung: **09.11.2022**
(21) Anmeldenummer: 22168996.1
(22) Anmeldetag: 20.04.2022
(51) Int. Cl.: F25D 23/12, C02F 1/00, G01N 33/18, F25D 29/00

(54) **KÜHL- UND/ODER GEFRIERGERÄT**

(30) Priorität: 04.05.2021 DE 102021111461; 09.06.2021 DE 102021114771
(71) Anmelder: Liebherr-Hausgeräte Lienz GmbH, 9900 Lienz (AT)
(72) Erfinder: MUIGG, Lukas, 9907 Tristach (AT)
(74) Vertreter: Herrmann, Uwe

(57) **Zusammenfassung**

Kühl- und/oder Gefriergerät mit einem Wassersystem, das einen Zulauf für Wasser zu dem Gerät oder ein Wasserreservoir, eine oder mehrere wasserführende Leitungen und eine oder mehrere Ausgabeeinrichtungen für Wasser oder auf Wasser basierende Produkte aufweist, wobei eine mit dem Wassersystem in Verbindung stehende Messvorrichtung vorhanden ist, die ausgebildet ist, den TOC-Gehalt und/oder den TC-Gehalt und/oder einen mit dem TOC-Gehalt bzw. dem TC-Gehalt korrelierten Wert des in dem Wassersytem befindlichen Wassers zu messen.

## Beschreibung

Die vorliegende Erfindung betrifft ein Kühl- und/oder Gefriergerät mit einem Wassersystem, das einen Zulauf für Wasser zu dem Gerät und/oder ein Wasserreservoir, eine oder mehrere wasserführende Leitungen und eine oder mehrere Ausgabeeinrichtungen für Wasser oder für auf Wasser basierenden Produkten, insbesondere Eis, aufweist.

Derartige Geräte sind aus dem Stand der Technik bekannt. Bei der Ausgabeeinrichung kann es sich beispielsweise um einen Dispenser für Wasser oder für Eiswürfel etc. handeln.

Aus dem Stand der Technik ist es bekannt, eine Minimierung eines etwaigen Keimwachstums bzw. die Einhaltung von Hygienevorschriften dadurch zu gewährleisten, dass die jeweils notwendigen (Bauteil-)Zertifizierungen eingehalten werden, welche auf ein minimales Wachstum von Organismen schließen lassen.

Bisher wurde in Kühlgeräten und/oder Kühl-Gefrierkombinationen keinerlei Rückschluss auf die Hygienesituation beispielsweise durch messtechnische Verfahren in den Wassersystemen des Gerätes getroffen. Grundlegend werden zwar die jeweils notwendigen (Bauteil-)Zertifizierungen eingehalten, welche auf ein minimales Wachstum von Organismen schließen lassen, jedoch ist aus unterschiedlichen Analysen bekannt, dass trotz Einhaltung dieser Zertifizierungen teilweise ggf. bevorzugte Bereiche für Mikroorgansimen gebildet werden.

Der vorliegenden Erfindung liegt die Aufgabe zugrunde, ein Kühl- und/oder Gefriergerät der eingangs genannten Art dahingehend weiterzubilden, dass ein Rückschluss auf eine etwaige Keimbelastung des in dem Gerät befindlichen Wassers bzw. des diesem zugeführten Wassers getroffen werden kann.

Diese Aufgabe wird durch ein Kühl- und/oder Gefriergerät mit den Merkmalen des Anspruchs 1 gelöst.

Danach ist vorgesehen, dass eine mit dem Wassersystem in Verbindung stehende Messvorrichtung vorhanden ist, die ausgebildet ist, den TOC-Gehalt und/oder den TC-Gehalt oder einen mit dem TOC-Gehalt oder dem TC-Gehalt korrelierten Wert des in dem Wassersytem befindlichen Wassers zu messen. Dadurch kann eine kontinuierliche oder diskontinuierliche Überwachung des Kohlenstoff-Gehaltes, im Folgenden als "C-Gehalt" bezeichnet, im Wassersystem bzw. in Teilen von diesen, wie z.B. in der Trinkwasserausgabe oder in dem Eisbereiter etc. des Kühl- und/oder Gefriergerätes erzielt werden. Das Wassersystem bzw. deren Komponenten können so auf die jeweils vorliegende "Biofilmmenge" überprüft werden.

Unter einer "mit dem Wassersystem in Verbindung stehenden Messvorrichtung" kann eine Messvorrichtung verstanden werden, die unmittelbar dem in dem Wassersystem befindlichen Wasser ausgesetzt ist, sowie aber auch eine Messvorrichtung, die nur mittelbar mit dem Wassersystem in Verbindung steht und der eine Probe aus dem Wassersystem zugleitet wird. Auch eine Kombination der beiden vorgenannten Optionen ist von der Erfindung umfasst.

Im Rahmen der vorliegenden Erfindung bedeuten:
TOC: Gesamter organischer Kohlenstoff ("Total organic carbon")
TC: Gesamter Kohlenstoff ("Total carbon")
TIC: Gesamter anorganischer Kohlenstoff ("Total inorganic carbon")

Eine Umsetzung bzw. eine solche Messung kann eine Inline-Messvorrichtung sein, d.h. eine Messvorrichtung, die ständig einen der vorgenannten Parameter misst.

Von der Erfindung ist auch eine diskontinuierliche Messung, d.h. eine von Zeit zu Zeit, ggf. in vorbestimmten Zeitabständen erfolgende Messung umfasst.

Denkbar ist es, dass die Messvorrichtung in einer wasserführenden Komponente angeordnet ist und dort die Messung vornimmt. Denkbar ist es auch, dass die Messvorrichtung einer Probenahmeeinrichtung ist, die von Zeit zu Zeit oder ständig Wasserproben nimmt.

Diese kann beispielsweise in einer Bypassleitung einer Leitung angeordnet sein, so dass ein Teil des Wassers stets durch die Bypassleitung strömt und dort die Messung erfolgt.

Von der Erfindung ist aber auch umfasst, dass diskontinuierlich eine Probe genommen und diese analysiert wird.

Vorzugsweise ist eine Auswerteeinrichtung vorhanden, die mit der Messvorrichtung derart in Verbindung steht, dass mittels der Messvorrichtung erhaltene Messdaten an die Auswerteeinheit übermittelbar sind.

Je nach Notwendigkeit kann die Auswerteeinheit ausgebildet sein, die Auswertung kontinuierlich oder diskontinuierlich vorzunehmen. Die Auswerteeinheit wird mit den vorzugsweise kontinuierlich, d.h. ständig gemessenen Messwerten beaufschlagt und nimmt dann darauf basierend die Auswertung vor.

Denkbar ist beispielsweise eine kontinuierliche Messung des TOC-Gehaltes. Die dabei gewonnenen Messwerte können kontinuierlich oder diskontinuierlich, z.B. in bestimmten Zeitabständen ausgewertet werden.

Aufgrund des Zusammenhangs zwischen "Biofilm" und der Verkeimungslage kann so ein Rückschluss auf die Hygiene in dem Wassersystem bzw. in einer oder mehreren Komponenten von diesem gezogen werden. Dieser Rückschluss kann dann - je nach Messwert - einen Impuls bzw. Auslöser für weitere Maßnahmen bilden, wie z.B. eine selbsttätige Spülung des Wassersystems oder von Teilen von diesem, einen Tausch des Wasserfilters des Gerätes sowie die Anzeige einer Reinigungsnotwendigkeit für den Nutzer.

Vorzugsweise ist die Auswerteeinheit ausgebildet, einen Vergleich zwischen dem aktuellen Messwert und einem Sollwert oder zwischen einem zeitlich gemittelten Messwert und einem Sollwert etc. durchzuführen. Basierend auf diesem Vergleich wird sodann in der Auswerteeinheit festgestellt, dass die Hygieneerfordernisse erfüllt sind oder dass eine Maßnahme, wie z.B. eine der vorgenannten Maßnahmen durchzuführen sind.

In einer Ausführungsform ist vorgesehen, dass die Messvorrichtung Mittel zur vollständigen UV-Oxidation des Wassers bzw. von deren Inhaltsstoffen sowie Mittel zur Messung der Leitfähigkeit des Wassers umfasst. Über die Messung der Leitfähigkeitsveränderung durch UV-Beaufschlagung des Wassers kann ein Rückschluss auf den TOC bzw. TC-Gehalt gezogen werden. Je größer die durch die UV-Beaufschlagung verursache Leitfähigkeitsänderung des Wassers ist, desto größer ist der TOC bzw. der TC Gehalt. Wird der TC, also der insgesamt vorliegende Kohlenstoffgehalt gemessen, kann der TOC durch Abzug des TIC vom TC-Wert bestimmt werden.

Die Messvorrichtung kann einen ersten Leitfähigkeitssensor stromaufwärts des Mittels zur UV-Oxidation und einen zweiten Leitfähigkeitssensor stromabwärts des Mittels zur UV-Oxidation aufweisen, wobei die Auswerteeinheit ausgebildet ist, die Differenz aus den durch die beiden Leitfähigkeitssensoren ermittelten Leitfähigkeitswerten zu bestimmen und darauf basierend den TOC- oder TC-Gehalt oder einen damit korrelierten Wert zu bestimmen. Basierend auf der auf diese Weise bestimmten Differenz kann dann in der Auswerteeinheit entschieden werden, ob die Hygienestandards eingehalten werden oder ob eine Maßnahme zur Hygieneverbesserung, wie z.B. ein Filtertausch notwendig ist.

In einer denkbaren Ausführung ist die Messvorrichtung an oder in der Ausgabeeinrichtung oder im Bereich der Ausgabeeinrichtung angeordnet. Dies ermöglicht einen Gesamtüberblick über den TOC-Gehalt oder dergleichen zu gewinnen, da die anderen mit Wasser beaufschlagten Komponenten, wie Behälter, Ventile etc. der Ausgabeeinrichtung vorgelagert sind.

Alternativ oder zusätzlich dazu ist es möglich, die Messvorrichtung dort anzuordnen, wo mit einer erhöhten Konzentration / Menge von TOC oder TC zu rechnen ist, wie z.B. an einem Aktivkohlefilter.

Auch weitere Anordnungen der Messvorrichtung sind denkbar und von der Erfindung mit umfasst.

Auch ist es möglich, dass an unterschiedlichen Positionen mehrere Messvorrichtungen vorhanden sind.

Das Wassersystem kann einen oder mehrere Filter zur Wasserreinigung aufweisen, wobei vorgesehen sein kann, dass die Messvorrichtung an oder in oder im Bereich des Filters angeordnet ist.

Wie ausgeführt, kann die Auswerteeinheit ausgebildet sein, in Abhängigkeit des oder der ausgewerteten Messdaten eine oder mehrere Maßnahmen durchzuführen oder zu veranlassen.

Eine mögliche Maßnahme besteht in der Ausgabe eines für den Nutzer des Gerätes optisch oder akustisch wahrnehmbaren Signals. Dieses Signal kann beispielsweise eine Textnachricht sein, aus der hervorgeht, dass der Filter gewechselt werden muss, dass das Wassersystem oder Teile von diesem gespült werden müssen etc.

Denkbar ist auch eine Maßnahme dahingehend, dass die Ausgabe von Wasser und/oder Eis blockiert wird, wenn zu hohe Werte an TOC oder TC festgestellt werden. Diese Blockade kann dann aufgehoben werden, wenn das Gerät feststellt oder der Benutzer bestätigt, dass eine bestimmte Maßnahme, wie z.B. ein Filterwechsel durchgeführt wurden.

Die Erfindung betrifft des Weiteren ein Verfahren zur Bestimmung des TOC-Gehalts und/oder des TC-Gehalts und/oder eines mit dem TOC-Gehalt bzw. dem TC-Gehalt korrelierten Wertes des in dem Wassersytem befindlichen Wassers eines Kühl- und/oder Gefriergerätes gemäß einem der Ansprüche 1 bis 10.

Vorzugsweise ist vorgesehen, dass der oder die Messwerte einer Auswerteeinheit zugeführt werden und basierend auf der in der Auswerteeinheit durchgeführten Auswertung keine oder eine oder mehrere Maßnahmen durchgeführt werden.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

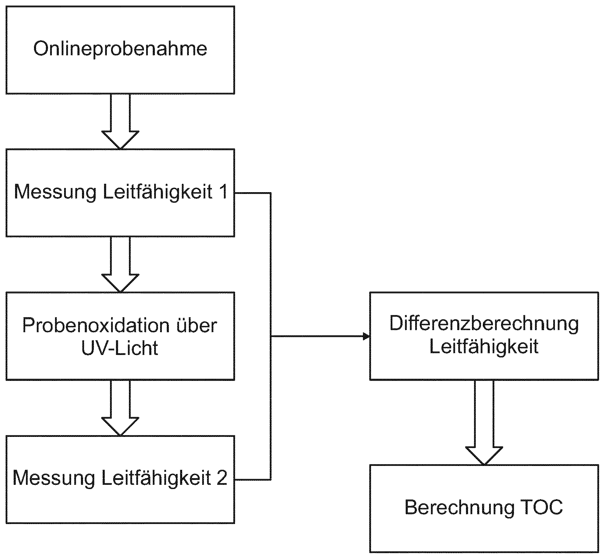

Weitere Einzelheiten und Vorteile der Erfindung werden anhand eines in der Zeichnung dargestellten Ausführungsbeispiels näher erläutert.

Die einzige Figur zeigt: eine Ausführung eines Funktionsprinzips eines Messvorgangs gemäß der Erfindung.

Gemäß der Figur erfolgt zunächst eine Online-Probenahme und sodann die Messung der Leitfähigkeit ("Leitfähigkeit 1").

Die Probe wird sodann mittels UV-Licht oxidiert und anschließend erfolgt eine erneute Messung der Leitfähigkeit dieser Probe ("Leitfähigkeit 2").

Der Auswerteeinheit werden diese beiden Leitfähigkeitsmesswerte zugeführt. Diese bildet aus den beiden Messwerten einen Differenzbetrag und ermittelt darauf basierend den TOC-Wert der Probe.

Basierend auf dem auf diese Weise ermittelten TOC-Wert wird sodann durch Vergleich mit einem Sollwert oder Sollwertbereich entschieden, ob die Hygienevorschriften eingehalten sind und keine weiteren Maßnahmen erforderlich sind oder ob Maßnahmen, wie z.B. ein Filteraustausch etc. vorzunehmen ist.

Vorteilhafte, optionale Merkmale und Aspekte der Erfindung werden im Folgenden dargestellt:
Grundlegend soll durch eine kontinuierliche und/oder diskontinuierliche Überwachung des C-Gehaltes in Wassersystemen von Kühlgeräten und/oder Kühlgefrierkombinationen die jeweils vorliegenden Komponenten, unabhängig der vorgesehenen Verwendung (Eisproduktion oder Trinkwasserausgabe), welche Einfluss auf die Trinkwasserhygiene haben, auf die jeweils vorliegende "Biofilmmenge" geprüft werden. Eine Umsetzung soll mittels Inlinemessung erfolgen, wo je nach Notwendigkeit eine kontinuierliche oder diskontinuierliche Auswertung erfolgen kann. Als Grundlage gilt hier die kontinuierliche Menge des TOC Gehaltes. Aufgrund des Zusammenhanges zwischen Biofilmthematik und Verkeimungslage kann so ein Rückschluss auf die Hygienethematik in den Wassersystemen geschlossen werden und somit ein Impuls für eine selbstständige Spülung, einen Tausch des Filters, sowie eine Reinigungsnotwendigkeit ausgelöst werden.

Das Wesentlich Schutzfähige an dieser Erfindung ist, dass mittels einer Messung des C-Gehaltes im Trinkwasser ein Rückschluss auf die Keimbelastung in den Trinkwassersystemen getroffen werden kann und dies als Ausgangslage für weitere Maßnahmen wie beispielsweise einen Spülvorgang, eine Reinigungsaufforderung, sowie einen Filtertausch vorgesehen werden kann.

Erfindungsgemäß kann vorgesehen sein, dass eine Messung des TOC Gehaltes grundsätzlich inline, kontinuierlich, jedoch nach Bedarf auch diskontinuierlich ausgewertet werden kann. Als kontinuierliches inline-Messprinzip kann grundsätzlich die Messung der Leitfähigkeitsveränderung über die direkte UV-Oxidation und somit Rückschluss auf den TOC Gehalt verwendet werden. Dieses Messprinzip ist in der Figur schematisch dargestellt. Grundsätzlich kann jedoch eine Messung des TC Wertes durchgeführt werden, sofern eine Kompensationsmöglichkeit über den TIC vorgesehen wird. Dies kann einerseits über eine kontinuierliches "mitloggern" erfolgen, andererseits kann jedoch die Möglichkeit über eine händische Kompensation erfolgen.

Vorzugsweise ist angedacht, dass die Onlineprobenahme entweder möglichst an der Ausgabeeinheit angebracht ist, sodass ein Gesamtüberblick erworben wird, andererseits kann angedacht sein, dass die Probenahme nach Bauteilen mit erhöhtem Einfluss (Aktivkohlefilter) bzw. Gefahr einer etwaigen Keimbelastung eingesetzt wird. Grundlegend werden jedoch andere Formen dadurch nicht ausgeschlossen.

## Patentansprüche

1. Kühl- und/oder Gefriergerät mit einem Wassersystem, das einen Zulauf für Wasser zu dem Gerät und/oder ein Wasserreservoir, eine oder mehrere wasserführende Leitungen und eine oder mehrere Ausgabeeinrichtungen für Wasser oder auf Wasser basierende Produkte aufweist, **dadurch gekennzeichnet, dass** eine mit dem Wassersystem in Verbindung stehende Messvorrichtung vorhanden ist, die ausgebildet ist, den TOC-Gehalt und/oder den TC-Gehalt und/oder einen mit dem TOC-Gehalt bzw. dem TC-Gehalt korrelierten Wert des in dem Wassersytem befindlichen Wassers zu messen.

2. Kühl- und/oder Gefriergerät nach Anspruch 1, **dadurch gekennzeichnet, dass** es sich um eine Inline-Messvorrichtung handelt.

3. Kühl- und/oder Gefriergerät nach Anspruch 1 oder 2, **dadurch gekennzeichnet, dass** eine Auswerteeinrichtung vorhanden ist, die mit der Messvorrichtung derart in Verbindung steht, dass mittels der Messvorrichtung erhaltene Messdaten aus die Auswerteeinheit übermittelbar sind.

4. Kühl- und/oder Gefriergerät nach Anspruch 3, **dadurch gekennzeichnet, dass** die Auswerteeinheit ausgebildet ist, die Auswertung kontinuierlich oder diskontinuierlich vorzunehmen.

5. Kühl- und/oder Gefriergerät nach einem der vorhergehenden Ansprüche, **dadurch gekennzeichnet, dass** die Messvorrichtung Mittel zur UV-Oxidation des Wassers sowie Mittel zur Messung der Leitfähigkeit des Wassers umfasst.

6. Kühl- und/oder Gefriergerät nach Anspruch 5, **dadurch gekennzeichnet, dass** die Messvorrichtung einen ersten Leitfähigkeitssensor stromaufwärts des Mittels zur UV-Oxidation und einen zweiten Leitfähigkeitssensor stromabwärts des Mittels zur UV-Oxidation aufweist und das die Auswerteeinheit ausgebildet, die Differenz aus den durch die beiden Leitfähigkeitssensoren ermittelten Leitfähigkeitswerten zu bestimmen und darauf basierend den TOC- oder TC-Gehalt oder einen damit korrelierten Wert zu bestimmen.

7. Kühl- und/oder Gefriergerät nach einem der vorhergehenden Ansprüche, **dadurch gekennzeichnet, dass** die Messvorrichtung an oder in oder im Bereich der Ausgabeeinrichtung angeordnet ist.

8. Kühl- und/oder Gefriergerät nach einem der vorhergehenden Ansprüche, **dadurch gekennzeichnet, dass** das Wassersystem ein oder mehrere Filter aufweist und dass die Messvorrichtung an oder in oder im Bereich des Filters angeordnet ist.

9. Kühl- und/oder Gefriergerät nach einem der vorhergehenden Ansprüche, **dadurch gekennzeichnet, dass** die Auswerteeinheit ausgebildet, in Abhängigkeit des oder der ausgewerteten Messdaten eine oder mehrere Maßnahmen durchzuführen oder zu veranlassen.

10. Kühl- und/oder Gefriergerät nach Anspruch 9, **dadurch gekennzeichnet, dass** die Auswerteeinheit eine Ausgabeeinheit aufweist und dass die Maßnahme in der Ausgabe eines optisch oder akustisch wahrnehmbaren Signals besteht.

11. Verfahren zur Bestimmung des TOC-Gehalts und/oder des TC-Gehalts und/oder eines mit dem TOC-Gehalt bzw. dem TC-Gehalt korrelierten Wertes des in dem Wassersytem befindlichen Wassers eines Kühl- und/oder Gefriergerätes gemäß einem der Ansprüche 1 bis 10.

12. Verfahren nach Anspruch 11, **dadurch gekennzeichnet, dass** der oder die Messwerte einer Auswerteeinheit zugeführt werden und basierend auf der in der Auswerteeinheit durchgeführten Auswertung keine oder eine oder mehrere Maßnahmen durchgeführt werden.
